(19) Europäisches Patentamt
European Patent Office
Office européen des brevets

(11) **EP 1 972 211 B1**

(12) **FASCICULE DE BREVET EUROPEEN**

(45) Date de publication et mention
de la délivrance du brevet:
**28.04.2010 Bulletin 2010/17**

(51) Int Cl.:
*A23L 3/01* *(2006.01)*          *A23L 3/02* *(2006.01)*
*A23L 3/32* *(2006.01)*          *A23L 2/48* *(2006.01)*
*A23L 2/50* *(2006.01)*

(21) Numéro de dépôt: **07005762.5**

(22) Date de dépôt: **21.03.2007**

(54) **Stérilisation de liquides dans des recipients hermétiquement fermés**

Sterilisierung von Flüssigkeiten in hermetisch verschlossenen Behältern

Sterilization of liquid in hermetically sealed containers

(84) Etats contractants désignés:
**AT BE BG CH CY CZ DE DK EE ES FI FR GB GR
HU IE IS IT LI LT LU LV MC MT NL PL PT RO SE
SI SK TR**

(43) Date de publication de la demande:
**24.09.2008 Bulletin 2008/39**

(73) Titulaire: **Opus Industry SA
1000 Lausanne (CH)**

(72) Inventeur: **Zavadtsev, Aleksandr
143965 Reutov (RU)**

(74) Mandataire: **Reuteler, Raymond Werner et al
Reuteler & Cie SA
Chemin de la Vuarpillière 29
1260 Nyon (CH)**

(56) Documents cités:
**EP-B1- 1 328 167     WO-A1-00/56179
US-A- 3 272 636     US-A- 4 695 472
US-A- 5 235 905     US-A1- 2004 084 381**

EP 1 972 211 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne un procédé de stérilisation de liquides contenus dans des récipients hermétiquement fermés, et un dispositif pour la mise en oeuvre du procédé.

Une des méthodes de stérilisation couramment exploitée en industrie est par autoclave, où l'on traite des récipients par paquets (en anglais « batches ») typiquement à une température entre 90 °C et 130°C pour plusieurs minutes, à des cadences de plusieurs milliers de récipients par heure. Toutefois, la stérilisation à ces températures peut altérer sensiblement les propriétés du produit traité ( couleur, goût, odeur, qualités biophysiques, biochimiques et autres). Dans un procédé conventionnel de stérilisation thermique, l'élévation de température s'effectue lentement et permet aux micro-organismes de s'adapter et de mieux résister à l'augmentation de température.

Des procédés visant à réduire le seuil de température nécessaire pour stériliser un liquide aqueux, par l'application de champs électriques, sont décrits dans les brevets US 4,695,472 et EP 1 328 167. Le procédé décrit dans US 4,695,472 ne concerne toutefois que la stérilisation de flux de liquide et ne peut pas être employé pour la stérilisation de bouteilles ou d'autres récipients remplis de liquide. L'amplitude du champ électrique proposé, appliqué sur une bouteille d'une dizaine de centimètres de diamètre, nécessiterait des tensions très élevées, difficiles à générer et à appliquer de manière homogène.

Dans EP 1 328 167, un procédé pour la stérilisation de bouteilles ou d'autres récipients remplis de liquide est décrit. Il est proposé de limiter la température de seuil $T_S$ de stérilisation en soumettant le produit simultanément à un échauffement par champ électrique et à l'action de vibrations ultrasoniques. Cette technologie s'avère cependant peu efficace en pratique, d'une part parce que différents micro-organismes ont des sensibilités différentes aux vibrations ultrasoniques, en fonction de la fréquence et de l'amplitude, d'autre part l'application homogène d'ultrasons dans tout le volume du récipient est difficile à effectuer.

Par ailleurs, avec les procédés de stérilisation par électroporation connus, il est difficile d'obtenir une bonne uniformité de traitement de récipients hermétiques contenant du liquide, en raison de la rapidité d'échauffement et la forme des récipients, entraînant des disparités de température et de champ électrique dans le volume de liquide à stériliser. Pour compenser ces disparités, afin d'assurer une destruction fiable et irréversible de micro-organismes dans tout le volume de liquide, on pourrait augmenter la température moyenne et/ou l'amplitude ou le temps d'application du champ électrique. Toutefois, la conséquence serait une altération accrue des propriétés du liquide.

Lors de l'échauffement, la pression à l'intérieur du récipient augmente et peut s'accompagner d'une déformation irréversible du récipient, notamment s'agissant des bouteilles ou d'autres récipients en matière plastique. Les procédés de stérilisation par champ électrique ont l'avantage de diminuer la température et le temps de stérilisation par rapport à un procédé de pasteurisation thermique conventionnel. Il y a toutefois un avantage à diminuer la température et le temps de traitement encore plus pour réduire les effets dus à l'accroissement de la pression intérieure.

Des dispositifs de compensation de pression dans le domaine de stérilisation de récipients à haute température sont décrits dans les brevets GB390768, US2909436, FR1436405 et FR2035678. Dans ces systèmes, la pression interne est compensée par la pression du liquide entourant le récipient, déterminé par la hauteur de la colonne de liquide dans lequel les récipients sont immergés. Ce liquide sert également à chauffer le contenu du récipient, rendant le procédé de stérilisation relativement lent, avec des conséquences néfastes sur l'altération des propriétés de l'aliment dans le récipient. De tels procédés ne sont également pas prévus pour, ni adaptés à, la stérilisation de bouteilles en PET ou d'autres récipients en matériaux plastiques dont la résistance au fluage diminue fortement à la température de pasteurisation thermique conventionnelle.

Un but de l'invention est de fournir un procédé de stérilisation ou de pasteurisation performant, efficace et fiable à des cadences industrielles, apte à stériliser ou à pasteuriser des liquides contenus dans des récipients hermétiques, y compris des récipients de tailles et de formes courantes dans le domaine alimentaire en plastique ou d'autres matériaux ne supportant pas des températures élevées. Un but est également de fournir un dispositif pour la mise en oeuvre d'un tel procédé.

Un autre but de l'invention est de fournir un procédé de stérilisation ou de pasteurisation apte à stériliser ou à pasteuriser, à des cadences industrielles, des liquides contenus dans des récipients hermétiques y compris des récipients de tailles et de formes courantes dans le domaine alimentaire, qui n'altère pas, ou qui altère peu, les propriétés du liquide.

Il est avantageux de fournir un procédé de stérilisation d'un liquide qui n'échauffe pas le liquide, même localement, au dessus de 70°C, mais de préférence pas au dessus de 65°C.

Des objectifs de l'invention sont réalisés par un procédé de stérilisation selon la revendication 1 et par un dispositif selon la revendication 16.

Le procédé de stérilisation de récipients hermétiquement fermés contenant un liquide à stériliser selon la présente invention, comprend le transport des récipients dans une zone de traitement où les récipients sont immergés dans un flux de fluide extérieur, l'échauffement en volume du liquide à stériliser par ondes électromagnétiques à un taux supérieur à 28°C par seconde jusqu'à une température de traitement T se situant entre 20°C et 66°C, l'agitation du récipient lors de l'échauffement du liquide, et selon la valeur de la température de traitement T, l'exposition du liquide à un champ électrique en impulsions immédiatement ou peu après l'échauffement du liquide, l'amplitude E du champ électrique en V/cm étant choisie de sorte que l'équation :

$$C(T) \leq \log(E+1) \leq B(T)$$

soit satisfaite pour les valeurs :

$$B(T) = -2{,}340 \cdot 10^{-5}\, T^3 + 1{,}290 \cdot 10^{-3}\, T^2 - 3{,}110 \cdot 10^{-2}\, T + 5{,}0$$

$$C(T) = -4{,}503 \cdot 10^{-5}\, T^3 + 2{,}888 \cdot 10^{-3}\, T^2 - 5{,}900 \cdot 10^{-2}\, T + 4{,}0$$

où T est la température de traitement en degrés Celsius.

De manière très surprenante, les inventeurs ont trouvé qu'en réchauffant le liquide très rapidement, à une vitesse supérieure à 28°C par seconde, le champ électrique à appliquer pour détruire les micro-organismes peut être fortement réduit par rapport aux procédés connus. Ainsi, à des valeurs de température de traitement de 64 à 66°C, l'amplitude du champ électrique peut même être nulle. En d'autres termes, si l'on chauffe le liquide en volume en toute partie à plus de 28°C par seconde, la pasteurisation efficace et fiable du liquide ne nécessite aucune exposition au champ électrique pour une température de traitement au-delà de 64°C, et pour des températures inférieures, la pasteurisation peut-être effectuée avec une exposition à un champ électrique d'amplitude bien inférieure à ce que l'on propose conventionnellement.

En raison de l'importance de la vitesse d'échauffement sur l'efficacité de pasteurisation, l'échauffement uniforme en volume est très important pour assurer que l'entièreté du volume du liquide soit soumise à un échauffement rapide. A cette fin, le liquide est agité ou turbulisé et le réchauffement en volume est effectué par ondes haute fréquence ou micro-ondes. L'échauffement par ondes HF ou micro-ondes permet d'obtenir un échauffement par agitation des molécules d'eau en minimisant l'échauffement ohmique par courant électrique, pour éviter des problèmes d'effet de « pinch » induisant des non-uniformités d'échauffement. Les fréquences de ces rayonnements sont de préférence de plus de 1000 kHz.

Le champ électrique de traitement par électroporation est de préférence alternatif et fourni par impulsions, la fréquence du champ alternatif se situant de préférence entre 100kHz et 1000kHz.

Pour la plupart des micro-organismes représentant un danger pour les produits alimentaires et notamment les boissons, mais aussi pour des produits pharmaceutiques et médicaux, le mécanisme d'adaptation du micro-organisme à l'accroissement de la température ne se réalise pas à des vitesses d'échauffement supérieures à 28°C par seconde pendant toute la durée du procédé d'échauffement.

Les contraintes thermiques sur les membranes des micro-organismes dues à l'augmentation très rapide de la température du liquide s'ajoutent aux contraintes dues aux effets du champ électrique alternatif, dont la fréquence est choisie pour faire osciller les effets de contrainte sur les membranes et par conséquent amplifier les contraintes locales maximales que ces membranes subissent. Cette combinaison permet de mieux concentrer l'énergie du champ électrique sur la destruction des micro-organismes par électroporation, en minimisant la perte d'énergie électrique en chaleur et donc la puissance électrique nécessaire pour la destruction irréversible des micro-organismes. Cela permet de traiter des plus grands volumes et de plus facilement éviter des problèmes de claquage et d'échauffement local pouvant altérer les propriétés du liquide à stériliser.

L'énergie calorifique totale apportée au liquide à traiter par ladite ou lesdites impulsion(s) de champ électrique peut avantageusement être très faible, notamment inférieure à 0,05 J/cm$^3$.

Un avantage de la présente invention est donc de pouvoir effectuer très rapidement et à des températures inférieures à 66°C, avec un champ électrique relativement faible, voir nul, des opérations de destruction ou d'électroporation collective irréversibles sur des cellules se trouvant en grande quantité dans une solution aqueuse, notamment se trouvant à l'intérieur d'un récipient hermétiquement fermé Dans ce cas, il s'est avéré possible de réaliser de manière irréversible la destruction des micro-organismes, tels que des moisissures et des levures à l'état végétatif et sous forme de spores, à des températures ne dépassant pas 65°C, pour des durées de procédé ne dépassant pas une à deux secondes.

Cela rend possible la stérilisation efficace et de longue durée de produits à base d'eau ou contenant de l'eau, notamment de boissons (telles que les eaux minérales plates, eaux aromatisées, thé, jus de fruit et produits dérivés, lait et produits dérivés, bière) enfermées dans des récipients en matière plastique, notamment en PET, dont la température maximum de stabilité thermique est de l'ordre de 70 °C.

L'échauffement en volume peut être réalisé par des ondes électromagnétiques à haute fréquence ou à micro-ondes. Un flux de fluide échauffé s'écoulant autour des récipients permet d'améliorer, par échange thermique convectif, l'obtention d'un champ de température uniforme à l'intérieur du récipient. En plus, il permet, en augmentant la pression statique au fur et à mesure de l'échauffement du récipient et de son contenu, de compenser l'augmentation de pression à l'intérieur du récipient liée à l'échauffement du produit et par conséquent d'éviter une déformation plastique du récipient.

Un échauffement rapide en volume du produit enfermé dans le récipient crée des disparités de température dues au fait que les propriétés diélectriques du matériau du récipient sont substantiellement différentes de celles du produit contenant de l'eau. Cela signifie que la densité de puissance développée dans le produit est bien supérieure à celle développée dans le matériau du récipient. A des vitesses d'échauffement supérieures à 30 °C par seconde, les différences de température peuvent atteindre plus de 10 °C, et les gradients plus de 1.000 °C par centimètre. Les non-uniformités s'amplifient dans les zones d'épaississement de la paroi du récipient, par exemple le goulot et le fond de la bouteille. C'est dans ces endroits qu'il peut y avoir un risque que le procédé de stérilisation soit incomplet.

Etant donné que l'échauffement des parois n'a lieu pratiquement que par conduction thermique et par convection, les non-uniformités du champ des températures sont réduites en intensifiant les échanges de chaleur par conductivité thermique et par convection, d'une part en agitant le récipient pendant son échauffement, d'autre part en immergeant le récipient dans un flux de fluide (liquide ou gaz) extérieur échauffé à une température égale ou légèrement supérieure ( par exemple de 1 à 2°C) à celle visée pour le liquide à l'intérieur du récipient.

La vitesse relative du flux de fluide par rapport aux récipients détermine l'intensité du flux de chaleur du fluide vers le récipient et la différence locale des températures entre le liquide et la paroi du récipient qui le contient. Par exemple, en immergeant des bouteilles en PET de ½ litre remplies de thé dans un flux d'eau à 67°C, en les échauffant par micro-ondes à raison de 28°C par seconde de 20°C à 65°C (en moyenne), pour une vitesse de passage des bouteilles dans la station de stérilisation de 0, 42 mètre par seconde et une vitesse de flux d'eau à 67°C de 1, 2 mètre par seconde, un champ uniforme de température (+/- 0,5°C) a été obtenu en près d'une seconde.

De préférence, le flux de fluide dans lequel sont immergés les récipients est turbulisé, ce qui permet d'agiter simultanément les récipients.

Avantageusement, on peut utiliser la même station de stérilisation pour échauffer le contenu des récipients et le flux de liquide extérieur.

De préférence, l'on applique le champ électrique alternatif après une pause de l'ordre d'une à deux secondes suivant l'étape d'échauffement. Cette pause sert à uniformiser les températures par conductivité thermique et par convection. Dans le procédé de stérilisation selon l'invention, l'échauffement du liquide peut avoir lieu simultanément avec l'impulsion ou les impulsions de champ électrique.

Il est avantageux d'espacer la zone d'action de l'impulsion thermique de celle de l'impulsion de champ électrique. Par exemple, on peut insérer une zone de transit entre les deux, où le champ électrique est nul ou négligeable et où le champ de température est uniformisé dans le volume du liquide de manière à ce que la différence de température entre les parties centrales et périphériques du liquide ne dépasse pas un degré. Le liquide à traiter traverse cette zone de transit pendant la pause mentionnée plus haut entre l'échauffement du liquide et l'application du champ électrique.

D'autres buts et caractéristiques avantageux de l'invention ressortiront des revendications et de la description détaillée présentée ci-dessous, à titre d'illustration, avec référence aux dessins annexés, dans lesquels :

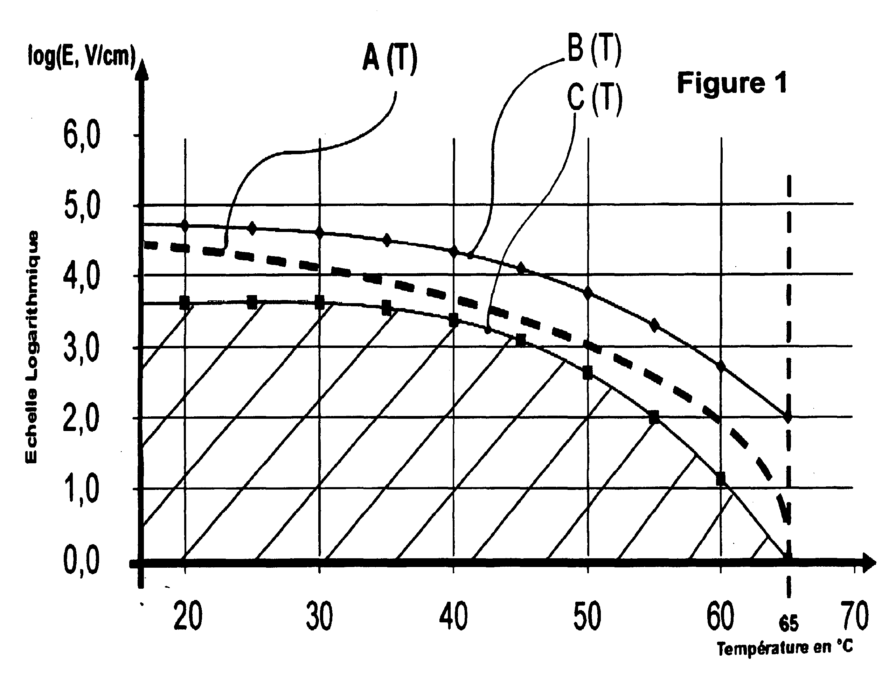

➢ La Figure 1 montre un graphique illustrant la relation entre la température de traitement et l'amplitude du champ électrique selon l'invention ;

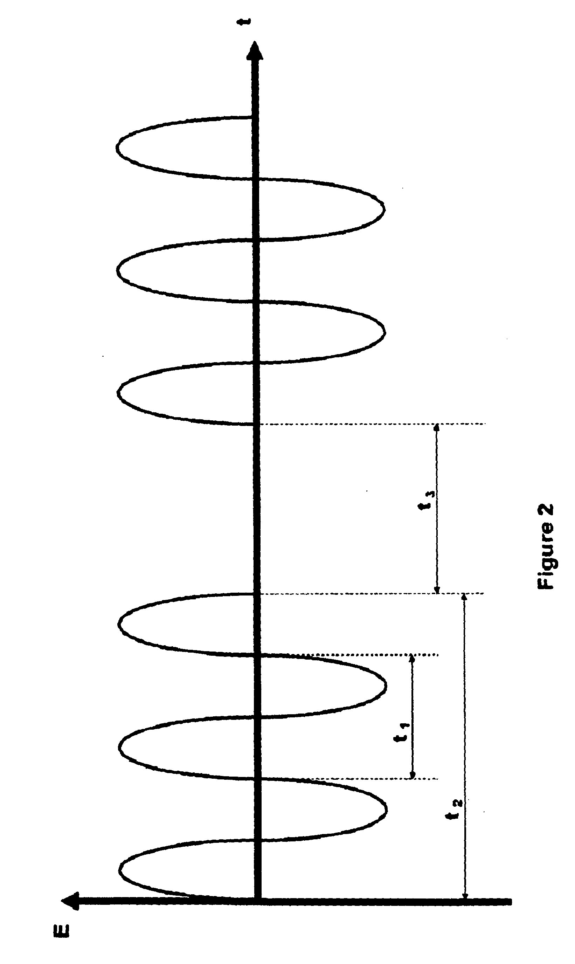

➢ La Figure 2 montre un graphique illustrant des impulsions de champ électrique selon l'invention;

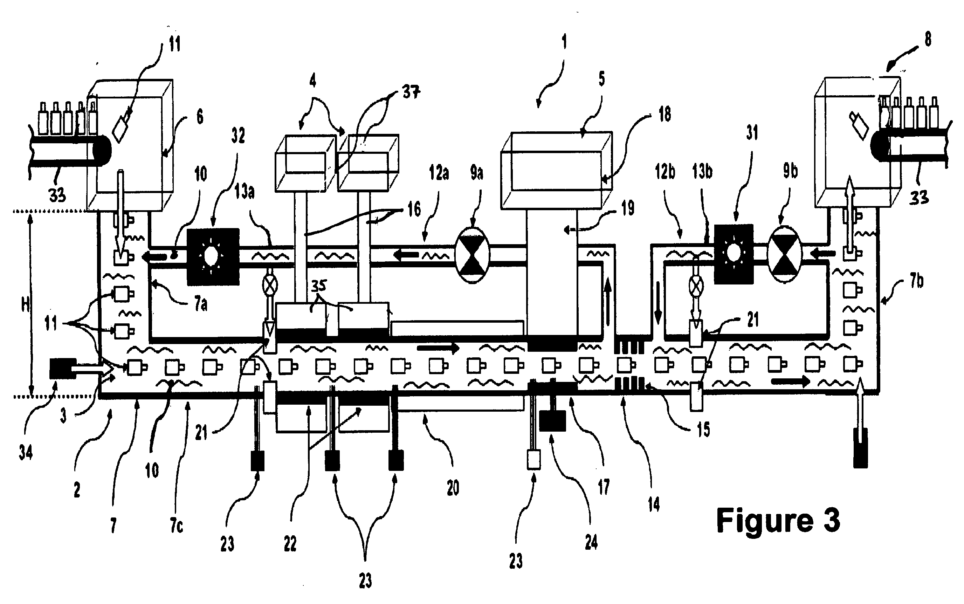

➢ La Figure 3 montre un dispositif pour la mise en oeuvre d'un procédé de stérilisation selon une forme d'exécution de la présente invention;

➢ La Figure 3 montre un dispositif pour la mise en oeuvre d'un procédé de stérilisation

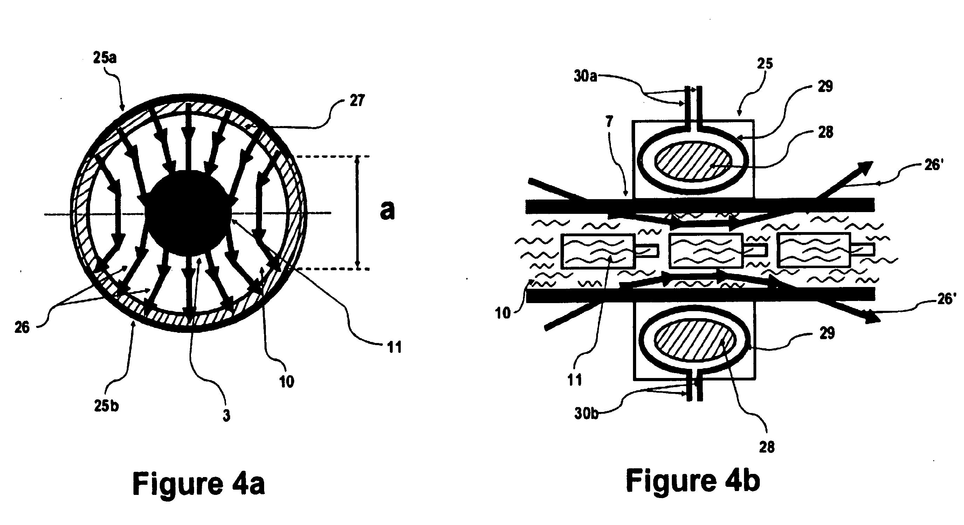

➢ La Figure 4a montre un dispositif distributeur de champ électrique selon un premier mode de réalisation ; et

➢ La Figure 4b montre un dispositif distributeur de champ électrique selon un second mode de réalisation.

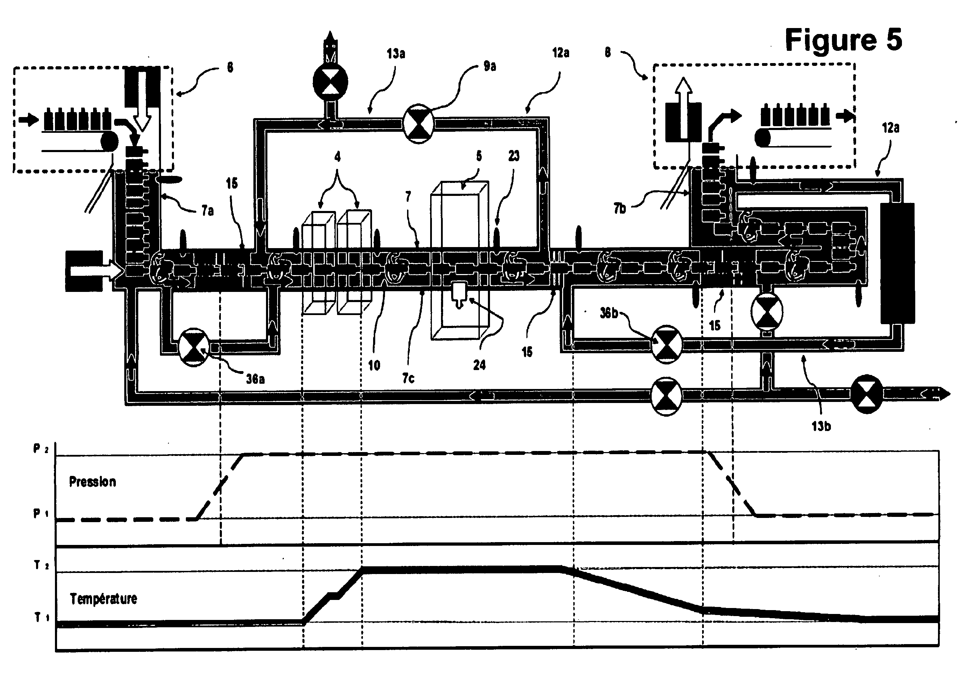

➢ La Figure 5 montre un dispositif pour la mise en oeuvre d'un procédé de stérilisation selon une autre forme d'exécution de la présente invention;

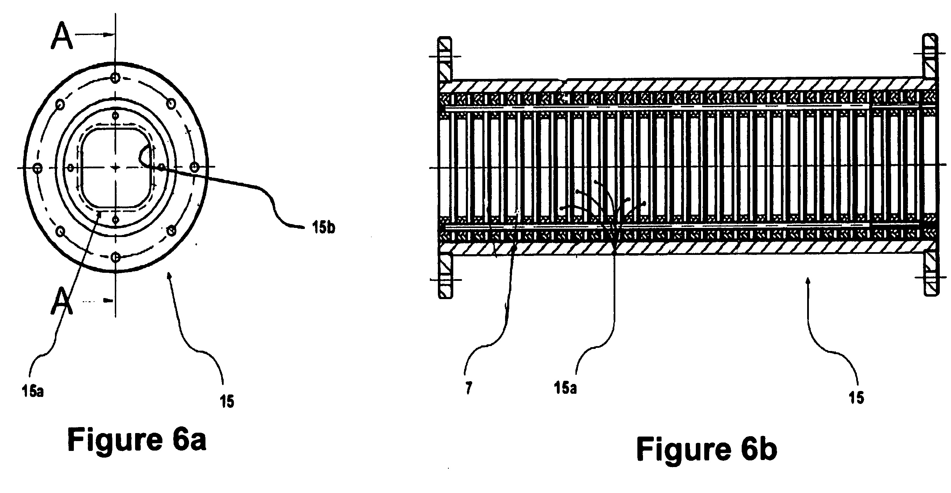

➢ La Figure 6a montre une partie du conduit comprenant un dispositif de joints (ici le cas de bouteilles de section non circulaire) ;

➢ La Figure 6b est une vue en coupe selon la ligne A-A de la figure 6a ; et

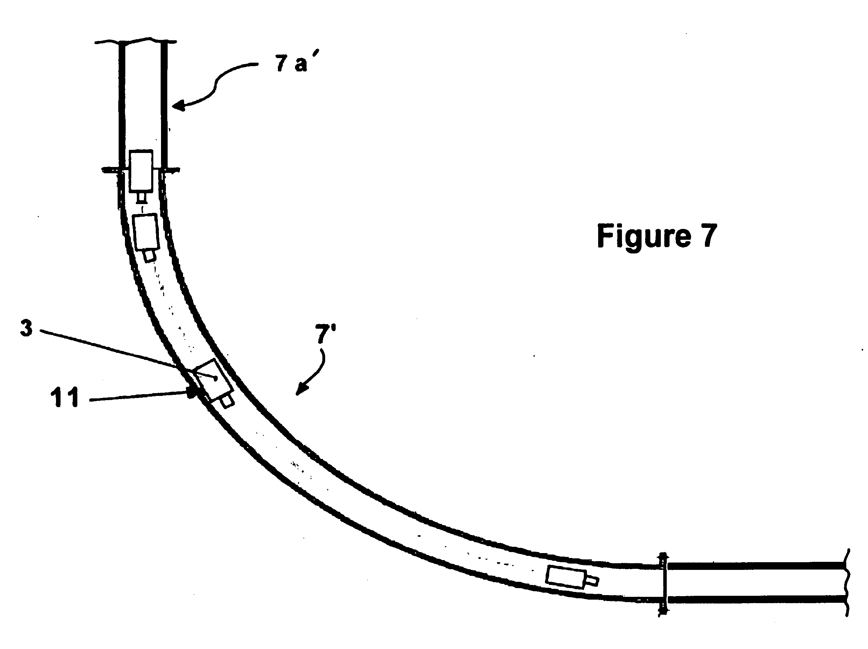

➢ La Figure 7 est une vue en coupe d'une partie de conduit de transport de récipients selon une variante de l'invention.

Le procédé de stérilisation selon la présente invention comprend le chauffage du liquide à traiter par un champ électrique ayant une fréquence supérieure à 1 MHz, à une vitesse supérieure à 28°C par seconde, jusqu'à une température de traitement T se situant entre 20°C et 66°C. Selon la valeur de la température de traitement T, le liquide est exposé à un champ électrique alternatif en impulsions immédiatement ou peu après l'échauffement du liquide, l'amplitude E du champ électrique en V/cm étant choisie de sorte que l'équation empirique:

$$C(T) \ \leq \ \log(E+1) \ \leq \ B(T)$$

soit satisfaite pour les valeurs :

$$B(T) = -2{,}340 \cdot 10^{-5} T^3 + 1{,}290 \cdot 10^{-3} T^2 - 3{,}110 \cdot 10^{-2} T + 5{,}0$$

$$C(T) = -4{,}503 \cdot 10^{-5} T^3 + 2{,}888 \cdot 10^{-3} T^2 - 5{,}900 \cdot 10^{-2} T + 4{,}0$$

où T est la température de traitement en degrés Celsius.

Cette relation est illustrée par le graphique de la figure 1.

B(T) représente la limite supérieure de l'amplitude du champ électrique raisonnablement nécessaire pour pasteuriser ou stériliser un liquide à base d'eau dans des conditions industrielles de pasteurisation de produits selon la présente invention.

C(T) représente la limite inférieure de l'amplitude du champ électrique au-dessous de laquelle il n'y a pas de destruction de tous les micro-organismes typiques représentant un danger pour la qualité et la conservation du produit ou pour la santé du consommateur ou de l'individu.

A(T) représente la limite inférieure de l'amplitude de champ électrique au-dessous de laquelle n'a pas lieu selon la présente invention la pasteurisation d'un produit à base d'eau et contenant les micro-organismes typiques représentant un danger pour la qualité et la conservation du produit ou pour la santé du consommateur ou de l'individu.

Par exemple, la valeur du champ électrique nécessaire pour pasteuriser un liquide suivant A(T) est :

$$E \approx 0 \text{ V/cm, quand } T = 65\ ^\circ C$$

$$E \approx 10^2 \text{ V/cm, quand } T = 60\ ^\circ C$$

$$E \approx 10^3 \text{ V/cm, quand } T = 50\ ^\circ C$$

$$E \approx 5 \cdot 10^3 \text{ V/cm, quand } T = 40\ ^\circ C$$

$$E \approx 10^4 \text{ V/cm, quand } T = 30\ ^\circ C$$

$$E \approx 5 \cdot 10^4 \text{ V/cm, quand } T = 20\,°C$$

On notera que cette relation ne donne qu'une première estimation, qui peut être précisée empiriquement en fonction des micro-organismes (des cellules) à détruire et des propriétés du liquide.

L'aspect de l'impulsion de champ électrique alternatif est illustré sur la Figure 2 où sont indiqués les temps $t_1$, $t_2$ et $t_3$.

L'oscillation du champ électrique est de préférence essentiellement sinusoïdale, mais peut être d'une autre forme.

Les caractéristiques et la forme des impulsions de champ électrique alternatif sont configurées pour maximiser l'électroporation des membranes des micro-organismes et réduire la génération de courant électrique perdu en chaleur. A cette fin, la période $t_1$ d'une oscillation du champ électrique a de préférence une valeur

$$t_1 > 1\ \mu s\ (10^{-6}\ \text{secondes})$$

Au-dessous de cette durée, les micro-organismes sont insensibles aux oscillations du champ électrique.

Pour une amplitude de champ électrique constante, plus grand est $t_1$, plus intense sont les pertes de courant dues à l'échauffement ohmique accompagnant le passage du courant électrique oscillant à travers le milieu échauffé, vu la résistivité électrique finie du milieu. Dans le cas de l'échauffement de conteneurs en matière plastique remplis de boisson par courants à haute fréquence, afin de minimiser ces pertes, il est très avantageux de limiter la fréquence à 100 kHz, soit $t_1$ à $10\,\mu s$, de préférence à $5\,\mu s$.

On a donc pour $t_1$ la condition limitante :

$$1\mu s < t_1 < 10\mu s.$$

La durée $t_2$ d'une impulsion de champ électrique oscillant est plus grande que la période $t_1$ d'une oscillation du champ électrique :

$$t_2 > t_1.$$

La valeur supérieure de $t_2$ est déterminée par l'échauffement total des zones de perturbations thermiques dues au fait que la résistance électrique des électrolytes - les boissons en sont un cas particulier - diminue avec l'augmentation de la température. Le courant électrique, dans ce cas, va toujours se concentrer dans des zones plus ou moins cylindriques orientées le long du vecteur de champ électrique. Ces zones, par la suite, se contractent rapidement, stimulées par les effets « pinch ». La température dans ces zones croît exponentiellement ce qui conduit à des échauffements locaux inacceptables, voire à des claquages.

Ces contraintes nous conduisent à la relation limitante pour $t_2$ :

$$t_2 < c \cdot dT \cdot R\ /\ E$$

où $c$, $dT$, $R$, $E$ sont, respectivement, la chaleur spécifique, l'écart limite de température, la résistivité du milieu, et l'amplitude du champ électrique.

Tenant compte du fait expérimental que la résistivité électrique d'un milieu aqueux telle qu'une boisson ne dépasse pas 10 Ohm.m et que $c = 4$ mégajoules$/ m^3$.degré, pour $dT < 5$degrés et $E = 1000$ kV/m, l'on a :

$$t_2 < 20\ \mu s.$$

La durée $t_3$ est le laps de temps entre deux impulsions de champ électrique. Elle est de préférence supérieure au temps de compensation des perturbations d'échauffement ohmique par les pulsations de turbulence hydrodynamique.
Si v est la vitesse caractéristique des instabilités hydrodynamiques et L est leur amplitude, la condition de compensation est :

$$t_3 > L/v$$

Pour le cas de la pasteurisation de bouteilles remplies de boisson et scellées, selon la présente invention, l'on a L > 0,003 m et v < 1m/s, d'où $t_3$ > 0,001 s.
La limite supérieure pour $t_3$ est donnée par la condition d'avoir au moins une impulsion par conteneur traité. Dans ce cas $t_3$ < LL/vv où LL est la dimension caractéristique du conteneur dans le sens de son mouvement à travers le champ électrique, et vv, sa vitesse.
Pour le cas typique de la pasteurisation de bouteilles de 0,5 l, LL = 0,3m et vv > 1 m/s, l'on a :

$$t_3 < 0,3 \text{ s.}$$

Si on traite un flux de liquide, $t_3$ < LLL / vvv où LLL est la longueur de la zone d'application du champ électrique et vvv, la vitesse de ce flux à travers cette zone.
Pour le cas typique où LLL = 0,3m et vvv > 1 m/s, l'on a :

$$t_3 < 0,3 \text{ s.}$$

Dans le procédé de stérilisation selon l'invention, l'échauffement du liquide peut avoir lieu simultanément avec l'impulsion ou les impulsions de champ électrique. En pratique, il est plus avantageux de soumettre d'abord le liquide à l'impulsion d'échauffement, et d'appliquer ensuite l'impulsion ou les impulsions de champ électrique. Cette pause est utile pour mieux uniformiser le champ de température dans le liquide à stériliser de manière à ce que toutes les zones du liquide, y compris celles des couches limites aux interfaces liquide-solide du récipient, acquièrent essentiellement la même température avant l'application du champ électrique.
Si x est l'épaisseur caractéristique de la couche limite (au plus 0,3 mm), la durée de pause tp est de préférence supérieure à :

$$t_p = (d.c.x^2)/z$$

où **d, c** et **z** sont respectivement la densité, la capacité thermique et la conductivité thermique du liquide à stériliser. Pour la plupart des applications, la durée de cette pause ne dépasse pas 1 ou 2 secondes.
Pour certaines applications il est avantageux d'espacer la zone d'action de l'impulsion thermique de celle de l'impulsion de champ électrique. Par exemple, on peut insérer une zone de transit entre les deux, où le champ électrique est nul ou négligeable et où le champ de température est uniformisé dans le volume du liquide de manière à ce que la différence de température entre les parties centrales et périphériques du liquide ne dépasse pas un degré. Le liquide à traiter traverse cette zone de transit pendant la pause mentionnée plus haut entre l'échauffement du liquide et l'application du champ électrique.
Les figures 3 et 5, illustrent schématiquement des dispositifs de mise en oeuvre du procédé selon différentes formes d'exécution de la présente invention.
Le dispositif 1 comprend un système de transport 2 du liquide à traiter 3, une station d'échauffement en volume 4 du liquide à traiter et une station d'application d'un champ électrique en impulsions 5.
Le système de transport 2 comprend une station d'entrée 6, un conduit de transport 7, et une station de sortie 8. Les récipients peuvent êtres amenés par un convoyeur standard 33 et déposés sur une chaîne à godets (ou tout autre mécanisme équivalent) dans une partie de colonne 7a du conduit 7.
Le système de transport peut en outre comprendre un système de pompage 9a, 9b, pour la circulation du liquide de transport 10 dans lequel des récipients hermétiques 11 contenant le liquide à traiter sont immergés. Le système de transport peut avantageusement comprendre un circuit chaud 12a et un circuit froid 12b, chacun muni d'un système de pompage 9a, 9b et de re-circulation du liquide de transport. Le circuit chaud transporte les récipients à travers les stations d'échauffement et d'application du champ électrique et retourne le liquide de transport par un conduit de retour 13a au conduit de transport 7 à proximité de la station d'entrée. Le circuit froid 13b a également un système de pompage 9b et un conduit de retour 13b interconnectant le conduit de transport 7 entre une position à proximité de la station de sortie 8 et une interface 14 séparant les circuits chaud et froid.

L'interface 14 comporte avantageusement un (ou plusieurs) dispositif(s) de joints 15 (voir figures 6a et 6b) comprenant une pluralité de parois flexibles ou élastiques 15a juxtaposées dans une section du conduit 7, par exemple en caoutchouc, comprenant des ouvertures 15b épousant le profil du récipient à traiter. Ainsi, les récipients participent à la création de l'étanchéité entre les circuits chaud et froid. Des dispositifs de joints 15 peuvent également être disposés en d'autres endroits le long du conduit de transport 7, par exemple en amont de la station d'échauffement 4.

Les circuits froid et chaud peuvent comprendre en outre des échangeurs de chaleur 31, 32 sur le conduit de retour, pour récupérer la chaleur du liquide de transport et/ou du liquide à traiter.

Le circuit froid permet de diminuer rapidement la température du liquide à traiter afin de préserver les propriétés du liquide et, le cas échéant, réduire les problèmes de déformation de récipients en matière plastique.

La station d'échauffement 4 comprend un système de génération d'impulsions thermiques 35 alimenté par un générateur d'énergie thermique 37. Le générateur thermique peut par exemple être sous forme d'un générateur de champ électrique haute fréquence opérant à une fréquence supérieure à 1 MHz ou d'un générateur de micro-ondes. L'énergie est transférée du générateur 37 au système 35 par l'intermédiaire d'un câble coaxial ou d'un guide d'ondes 16. Il est possible de prévoir plusieurs générateurs disposés de manière juxtaposée le long du conduit de transport 7.

La station d'application d'un champ électrique 5 comprend un distributeur d'impulsions de champ électrique oscillant bipolaire 17 relié à un générateur d'impulsions de champ électrique oscillant bipolaire 18 par l'intermédiaire d'un câble coaxial 19. Il convient de souligner que, comme évoqué précédemment, pour des températures de traitement supérieures à 64°C, il est possible de se dispenser de la station d'application de champ électrique.

Les stations d'impulsion thermique 4 et d'application du champ électrique 5 sont séparées par une section de transit du conduit thermiquement isolée 20 créant une pause entre le traitement thermique et le traitement d'impulsions électriques. Cette pause permet avantageusement d'uniformiser le champ de température dans le liquide à traiter et sur les surfaces des corps solides à son contact.

Dans l'exemple de réalisation de la figure 3, le liquide à stériliser est contenu dans des récipients 11 immergés dans un liquide de transport 10 s'écoulant dans le conduit 7 pour transporter les récipients. Les récipients peuvent par exemple être des bouteilles en plastique, remplies par exemple d'une boisson ou d'un aliment liquide.

Il est également possible de transporter les récipients contenant le liquide à stériliser à travers un station d'échauffement et une station d'application du champ électrique par des moyens autres qu'un liquide dans un conduit, par exemple par un flux de gaz sous pression dans un conduit (la pression du gaz étant choisie de manière à compenser la pression à l'intérieure du conteneur ce qui permet d'éviter toute déformation du conteneur due à l'échauffement) ou par un mécanisme de transport mécanique tel qu'un système convoyeur. Néanmoins, un système de transport par fluide a l'avantage de permettre une bonne uniformité dans la distribution de température autour du récipient lors de son échauffement et pendant la pause avant l'application du champ électrique. L'utilisation d'un liquide de transport ayant des propriétés diélectriques similaires à celles du liquide à stériliser permet avantageusement de bien maîtriser l'échauffement du liquide à stériliser ainsi que l'application du champ électrique local dans le liquide à stériliser.

Les récipients, en matière diélectrique, peuvent êtres sous forme de conteneurs rigides, tels que des bouteilles en verre ou en plastique (par exemple PET ou encore d'autres polymères).

Un ou plusieurs dispositifs d'agitation 21 peuvent être ajoutés au système pour agiter le liquide de transport et les récipients se trouvant dans le liquide de transport. Dans une variante, le dispositif d'agitation comprend un ou plusieurs jets (buses) (non illustrés) disposé(s) sur la paroi du conduit et débouchant à l'intérieur du conduit, pour injecter un fluide afin de créer des turbulences dans le fluide de transport s'écoulant dans le conduit, uniformisant ainsi le champ de température dans le liquide. Des récipients transportés dans le conduit peuvent également être agités ou mis en rotation, par exemple par un contrôle de courants en vortex dans le liquide de transport, afin d'uniformiser le liquide à traiter à l'intérieur des récipients. Des dispositifs d'agitation 21 peuvent également être disposés dans la partie de circuit froid 12b pour accélérer le refroidissement du liquide dans le récipient après le traitement de stérilisation ou de pasteurisation.

Des tubulures en matière diélectrique (du quartz, par exemple) 22 sont montées dans le conduit de manière à assurer le passage du champ électrique servant à l'échauffement du liquide à l'intérieur du conduit.

Des capteurs de température 23 sont disposés tout le long du conduit pour la mesure de la température du liquide à l'entrée de la station de génération d'impulsions thermiques, dans la zone d'échauffement, à la sortie de cette zone et à la sortie de la section de transit 20 du conduit.

Un capteur de champ électrique 24 est disposé dans la zone d'application du champ électrique.

Dans une forme d'exécution du dispositif, un mécanisme est prévu pour assurer une vitesse variable du déplacement des corps solides pendant leur passage dans le conduit, par exemple en changeant la section (diamètre) du conduit afin de varier la vitesse du flux du liquide de transport.

Un dispositif distributeur de champ électrique, selon une première variante, est représenté à la figure 4a. Dans cette variante, le distributeur comporte des électrodes 25a, 25b disposées de part et d'autre du conduit pour assurer le passage d'impulsions de champ électrique alternatif de fréquence entre 100kHz et 1000 kHz transversalement à travers le conduit 7 (de la Figure 3), comme illustré par les lignes de champ 26.

En particulier, le champ électrique passe de l'électrode supérieure 25a à l'électrode inférieure 25b, les deux électrodes étant montées à l'intérieur d'un tube 27 (en quartz, par exemple), hermétiquement intégré dans le conduit. La distance « a » entre les électrodes peut être optimisée empiriquement de manière à assurer la meilleure uniformité possible du champ électrique transversal dans le volume des conteneurs 11. Si la distance a est par exemple de l'ordre de 4 cm, pour obtenir une amplitude de champ électrique effectif de 1 - 3 kV/cm, il faut avoir une différence de potentiel entre les électrodes de l'ordre de 400 -1200 kV.

La Figure 4b illustre un dispositif distributeur de champ électrique selon une deuxième variante. Dans cette variante, les impulsions du champ électrique sont créées par un système à induction et les lignes de champ électrique 26' sont essentiellement longitudinales. Le conduit 7, rempli d'eau comme liquide de transport 10 transportant des récipients 11, tels que des bouteilles contenant un liquide à stériliser, traverse un corps du système à induction 25. Le dispositif distributeur de champ électrique est doté d'un noyau 28 et d'un ou de plusieurs enroulements primaires 29 raccordés à une alimentation par des connexions 30a, 30b. La quantité d'enroulements primaires peut être déterminée empiriquement, par exemple en mesurant le champ électrique présent dans le liquide de transport.

Dans la forme d'exécution de la figure 3, les récipients 11 sont immergés à une profondeur H dans une partie de colonne 7a du conduit de transport 7 remplie du liquide de transport 10.

La colonne de liquide de transport exerce une pression extérieure qui tend à compenser la pression intérieure lors de l'échauffement du liquide à traiter selon la formule (2) qui permet de déterminer la hauteur H de la colonne correspondant à la température $T > T_1$.

$$(2) \quad H \times d \times g = (T_2 / T_1) \times P_1 - C + V_P + V_S$$

où:

« **H** » est la hauteur de la colonne de liquide dans laquelle sont immergés les récipients à traiter ;

« **d** » est la densité du liquide extérieur ;

« **g** » est l'accélération locale de la pesanteur ;

« $P_0$ » est la pression initiale du liquide compressible dans le récipient à son entrée dans le dispositif ;

« $V_S$ » est la différence entre les pressions des vapeurs saturées du liquide incompressible aux températures $T_2$ et $T_1$. Pour l'eau, à $T_1$=20°C par exemple, la pression des vapeurs saturées est très petite et, pratiquement, $V_S$ est égale à la pression des vapeurs saturées de l'eau à la température $T_2$. Par exemple, si $T_2$=65°C, alors $V_S$=0,25 bar ;

« **C** » est égal à $(k \times V_V)$ où **k** est le coefficient d'élasticité volumique du matériau du récipient à la température $T_2$ et $V_V$ est la déformation volumique ;

« $V_P$ » est la variation de pression intérieure due à la variation de saturation du liquide incompressible par le liquide compressible. $V_P$ se mesure dans un récipient non déformable (par exemple en verre) de même forme et volume que le récipient traité, comme la différence de pression entre la pression manométrique réelle à la température $t_2$ et la pression $P_2 = P_0 \times (T_2 / T_1)$. Pour des boissons non saturées de $CO_2$, comme par exemple les eaux aromatisées ou le lait, $V_p$ est proche de zéro. La compensation est totale quand **C** = 0.

On peut diminuer la profondeur H en augmentant la densité d du milieu liquide extérieur utilisé dans lequel sont plongés les récipients. En particulier, on peut ajouter à ce liquide des corps solides de petite dimension p (p doit être de beaucoup inférieur à la dimension caractéristique du récipient), mais de densité supérieure à celle du liquide, par exemple sous forme de poudre. Cette mesure ne sera efficace que lorsque la pression exercée par les corps solides sera égale dans toutes les directions. Il faudra pour cela que les corps solides soient pourvus d'un mouvement chaotique dont la vitesse moyenne sera supérieure à la racine carrée de **gp** où :

« **g** » est l'accélération locale de la pesanteur

« **p** » est la dimension des corps solides

et que leur quantité spécifique n (quantité de corps solide par unité de volume) corresponde à l'augmentation de densité d visée.

Pour satisfaire à cette condition, il faut que la force de pesanteur du corps solide de masse m, soit mg, soit inférieure à la force **F** exercée par ce corps sur toute paroi suite à son inertie. Si **v** est la vitesse de mouvement chaotique, on peut obtenir pour **F** l'ordre de grandeur suivant : **F = m x (v / t),** où **t = d / v,** alors **F = (mv²) / d.** Il faut donc que **F>>mg,** donc que **v>>(gd)**$^{(1/2)}$.

Si des bouteilles sont traitées séquentiellement et dans le sens de leur longueur, les unes derrière les autres, un poussoir 34 envoie les bouteilles dans la partie de conduit horizontal 7c.

Une fois remontées dans la partie de colonne de sortie du conduit 7b, les récipients peuvent êtres évacués par un poussoir ou autre mécanisme sur un convoyeur 33.

Dans la variante illustrée dans la figure 7, le conduit de transport 7' est sous forme d'un tube configuré pour pouvoir insérer des bouteilles dans le sens de leur longueurs dans la partie d'entrée 7a' du conduit, et les guider ainsi jusqu'à la sortie du conduit dans la partie de circuit froid. A cet effet, le tube comporte des rayons de courbure suffisamment large pour assurer la transition entre les parties de conduit verticales et horizontale. La circulation du liquide de transport dans le sens du mouvement des récipients facilite le déplacement des récipients le long du conduit, non seulement en raison de la pression exercée dans le sens du mouvement, mais aussi en raison de la portance (force d'Archimède) et de la lubrification créées par la présence du liquide autour des récipients.

Le fluide de transport peut toutefois aussi être un gaz sous pression, séparé de l'environnement d'où proviennent les récipients par deux sas mécaniques ou par deux sas où la pression varie de manière progressive afin de compenser les différences de pressions intérieure et extérieure et d'éliminer ainsi les déformation du conteneur, notamment lors du refroidissement du liquide dans les récipients. En somme, dans ce cas particulier, les zones d'immersion de hauteur **H** et de densité **d** sont remplacées par des sas qui assurent le passage des récipients de l'environnement d'où ils proviennent dans une zone sous pression, cette pression **P$_x$** étant égale à la pression intérieure **P$_i$** se développant dans le récipients lors de son échauffement.

En faisant référence à la forme d'exécution de la figure 5, afin de compenser la pression développée dans le récipient lors de son échauffement, l'on peut réduire la hauteur de la partie de colonne verticale du conduit en générant une pression dans la partie de conduit 7c traversant les stations de traitement 4, 5 et le circuit de refroidissement, par des pompes 36a, 36b injectant le gaz ou le liquide de transport dans ladite partie de conduit 7c. Des dispositifs de joints 15 tels que décrit précédemment sont disposés de part et d'autre de la section de conduit 7c sous pression.

Des manomètres peuvent être disposés sur tout le circuit pour contrôler la pression dans le conduit, et l'on peu aussi prévoir des soupapes de purge pour éliminer l'air du système ou pour évacuer le liquide du conduit.

Des sas mécaniques permettant le passage des récipients et séparant une zone de liquide extérieur au récipient qui est chaude d'une zone où ce liquide est froid, ou tout autre système de sas ou système classique servant de barrière à la pression mais autorisant le passage des récipients, peuvent remplacer les dispositifs de joints 15.

**Exemples :**

1. Décontamination de bouteilles scellées PET de 0,5 l et remplies de jus d'orange fraîchement pressé et contaminé avec des microorganismes « *Byssochlamys nivea* ». Le traitement a été effectué sur un dispositif du type illustré à la Figure 3:

➢ Concentration initiale des microorganismes : de 3,6 à 4,2 10⁵ unités/ml ;
➢ Quantité de bouteilles traitées pour chaque régime : 10 ;
➢ Température initiale : 20°C ;
➢ Durée de traitement : 3s (passage dans le conduit horizontal) ;
➢ Echauffement: micro-onde 1 GHz, puissance 180 kW (35°C/s) et 45 kW (9°C/s);
➢ Application du champ électrique :

■ Fréquence d'oscillation du champ électrique : 180 kHz ;
■ Durée d'un paquet d'oscillations : env. 0,02 ms ;
■ Fréquence des paquets d'oscillations : 15 Hz ;
■ $t_1 = 6\mu s$, $t_2 = 20\mu s$, $t_3 = 0,05s$ ;
■ Quantité d'impulsions : 12 pour 180 kW et respectivement 35 et 48 impulsions pour 45kW ;

➢ Productivité, vitesse linéaire des bouteilles : 0,4 m/s pour 180 kW et 0 ,1 m/s pour 45 kW. Longueur de la zone d'application du champ : 0,3 m ; durée d'application des impulsions de champ électrique : 0,75s ;

Résultats :

| Champ électrique (V/cm) | Vitesse de croissance de la température en °C/s | Température de traitement en °C, +/- 1°C | Concentration résiduelle après les tests (unités/ml) | Concentration résiduelle 2 mois après les tests (unités/ml) |
|---|---|---|---|---|
| 0 | 9 | 80 | <1 | <1 dans 80% des cas |
| 0 | 9 | 65 | De 5 à 20 | - |
| 0 | 35 | 65 | <1 | <1 dans 100% des cas |
| 0 | 35 | 62 | De 120 à 1500 | - |
| 30 | 35 | 62 | <1 | <1 dans 95% des cas |
| 0 | 35 | 60 | env.$10^4$ | - |
| 100 | 35 | 60 | <1 | <1 dans 100% des cas |
| 0 | 35 | 55 | env. 3-4.$10^5$ | - |
| 600 | 35 | 55 | <1 | <1 dans 100% des cas |

2. Décontamination sélective de bouteilles PET de 0,5 l, remplies de jus de pomme et contaminées avec des levures *Saccharomyces cerevisiae* et des moisissures *Aspergillus Niger.* Le traitement a été effectué sur un dispositif du type illustré à la Figure 2 :

➢ Concentration initiale *Saccharomyces cerevisiae :* 1,2 - 3,1. $10^5$ unités/ml ;
➢ Concentration initiale *Aspergillus niger :* 1,5 - 4,2. $10^5$ unités/ml ;
➢ Quantité de bouteilles traitées pour chaque régime : 10 ;
➢ Température initiale : 20°C ;
➢ Durée de traitement : 3s (passage dans le conduit horizontal) ;
➢ Echauffement: micro-onde 1 GHz, puissance 180 kW (35°C/s) et 45 kW (9°C/s);
➢ Application du champ électrique :

■ Fréquence d'oscillation du champ électrique : 180 kHz ;
■ Durée d'un paquet d'oscillations : env. 0,02 ms ;
■ Fréquence des paquets d'oscillations : 15 Hz ;
■ $t_1$ = 6$\mu$s, $t_2$ = 20$\mu$s, $t_3$ = 0,05s ;
■ Quantité d'impulsions : 12 pour 180 kW et respectivement 35 et 48 impulsions pour 45kW ;

➢ Productivité, vitesse linéaire des bouteilles : 0,4 m/s pour 180 kW et 0 ,1 m/s pour 45 kW. Longueur de la zone d'application du champ : 0,3 m ; durée d'application des impulsions de champ électrique : 0,75s ;
➢

Résultats :

| Champ électrique (V/cm) | Vitesse de croissance de la température en °C/s | Température de traitement en °C, +/- 1°C | Conc. résiduelle après les tests (unités/ml) *Sacch.cer.* | Conc. résiduelle après les tests (unités/ml) *Asp. niger* |
|---|---|---|---|---|
| 0 | 9 | 70 | 2,8 .$10^1$ | 5.$10^2$ |

(suite)

| Champ électrique (V/cm) | Vitesse de croissance de la température en °C/s | Température de traitement en °C, +/- 1°C | Conc. résiduelle après les tests (unités/ml) *Sacch.cer.* | Conc. résiduelle après les tests (unités/ml) *Asp. niger* |
|---|---|---|---|---|
| 0 | 35 | 70 | <1 | <1 |
| 0 | 9 | 65 | $1,5 .10^3$ | $1,8.10^3$ |
| 0 | 35 | 65 | <1 | <1 |
| 65 | 9 | 60 | $5,2 .10^1$ | $3,7.10^1$ |
| 65 | 35 | 60 | <1 | <1 |
| 120 | 9 | 60 | 3-5 | 6-8 8 |
| 120 | 35 | 60 | <1 | <1 |
| 120 | 9 | 50 | $3,2. 10^4$ | $2,2.10^3$ |
| 120 | 35 | 50 | $7,2. 10^1$ | $5-6.10^1$ |
| 1020 | 9 | 50 | $2,7.10^2$ | $1,0.10^2$ |
| 1020 | 35 | 50 | <1 | <1 |
| 2540 | 9 | 45 | 3 -5 | $1,1 .10^1$ |
| 2540 | 35 | 45 | <1 | <1 |

**Revendications**

1. Procédé de stérilisation ou de pasteurisation d'un liquide à traiter contenu dans des récipients hermétiquement fermés, comprenant le transport des récipients dans une zone de traitement où les récipients sont immergés dans un flux de fluide de transport extérieur, le chauffage en volume du liquide à traiter à une vitesse supérieure à 28°C par seconde, jusqu'à une température de traitement **T** se situant entre 20°C et 66°C, l'agitation du récipient lors de l'échauffement du liquide, et selon la valeur de la température de traitement **T,** l'exposition du liquide à un champ électrique de traitement par électroporation immédiatement ou peu après l'échauffement du liquide, l'amplitude **E** du champ électrique en V/cm étant choisie de sorte que l'équation :

$$C(T) \ \leq \ \log (E+1) \ \leq \ B(T)$$

soit satisfaite pour les valeurs :
$B(T) = -2,340 . 10^{-5} T^3 + 1,290 . 10^{-3} T^2 - 3,110 . 10^{-2} T + 5,0$
$C(T) = -4,503 . 10^{-5} T^3 + 2,888 . 10^{-3} T^2 - 5,900 . 10^{-2} T + 4,0$
où T est la température de traitement en degrés Celsius.

2. Procédé selon la revendication 1, **caractérisé en ce que** le champ électrique de traitement par électroporation est alternatif avec une fréquence d'oscillation se situant entre 100kHz et 1000 kHz, et est fourni en impulsions.

3. Procédé selon la revendication 2 **caractérisé en ce que** l'énergie calorifique totale apportée au liquide à traiter par ladite ou lesdites impulsions de champ électrique est inférieure à 0,05 J/cm$^3$.

4. Procédé selon la revendication 2 ou 3 **caractérisé en ce que** la durée d'application d'une impulsion du champ électrique est comprise entre 10 et 100 microsecondes et la fréquence de répétition des impulsions de champ électrique se situe entre 10 et 100 Hz.

5. Procédé selon l'une quelconque des revendications précédentes, **caractérisé en ce que** l'application du champ électrique de traitement par électroporation est effectuée après l'étape de chauffage du liquide suivie d'une pause pendant laquelle le champ électrique est nul ou négligeable.

6. Procédé selon l'une quelconque des revendications précédentes, **caractérisé en ce que** la vitesse d'échauffement est supérieure à 30°C par seconde.

7. Procédé selon l'une quelconque des revendications précédentes, **caractérisé en ce que** le fluide de transport est de l'eau ou un liquide à base d'eau.

8. Procédé selon la revendication 7, **caractérisé en ce que** le liquide de transport est turbulisé en rotation autour des récipients.

9. Procédé selon l'une quelconque des revendications précédentes **caractérisé en ce que** les pressions statiques développées dans les zones de traitement sont créées par des systèmes de pompage et de sas.

10. Procédé selon l'une quelconque des revendications précédentes, **caractérisé en ce que** les pressions statiques développées dans la zone de traitement sont créées par des colonnes de liquide constituant le fluide extérieur surplombant la zone de traitement des récipients.

11. Dispositif pour la mise en oeuvre d'un procédé de stérilisation ou de pasteurisation d'un liquide à traiter à base d'eau ou contenant de l'eau contenu dans des récipients hermétiquement fermés, comprenant un système de transport (2) du liquide à traiter (3), une station d'échauffement en volume (4) du liquide à traiter comportant un générateur d'ondes opérant à une fréquence supérieure à 1MHz et une station d'application d'un champ électrique en impulsions (5), le système de transport comprenant un conduit de transport (7, 7') dans lequel circule un flux de fluide de transport, et des moyens d'agitation ou de turbulisation du fluide de transport à proximité de la station d'échauffement, le système d'échauffement étant configuré pour échauffer le liquide dans les récipients traversant la station d'échauffement à une température de traitement T se situant entre 20°C et 66°C à un taux supérieur à 28°C par seconde, et la station d'application d'un champ électrique en impulsions (5) étant configurée pour générer un champ électrique de traitement par électroporation immédiatement ou peu après l'échauffement du liquide, d'amplitude E en V/cm de sorte que l'équation :

$$C(T) \ \leq \ \log(E+1) \ \leq \ B(T)$$

soit satisfaite pour les valeurs :

$B(T) = -2,340 \cdot 100^{-5} T^3 + 1,290 \cdot 10^{-3} T^2 - 3,110 \cdot 10^{-2} T + 5,0$
$C(T) = -4,503 \cdot 10^{-5} T^3 + 2,888 \cdot 10^{-3} T^2 - 5,900 \cdot 10^{-2} T + 4,0$ où T est la température de traitement en degrés Celsius.

12. Dispositif selon la revendication précédente **caractérisé en ce que** la station de génération de champs électriques par impulsions est configurée pour générer un champ électrique alternatif avec une fréquence d'oscillation se situant entre 100kHz et 1000 kHz en impulsions avec une durée comprise entre 10 et 100 microsecondes.

13. Dispositif selon la revendication 12 **caractérisé en ce que** le système de génération de champ électrique par impulsions est configuré pour apporter une énergie calorifique totale inférieure à 0,05 J/cm$^3$ au liquide à traiter.

14. Dispositif selon l'une quelconque des revendications 11 à 13, **caractérisé en ce que** le fluide de transport est de l'eau ou un liquide à base d'eau.

15. Dispositif selon l'une quelconque des revendications 11 à 14, **caractérisé en ce que** le système de transport comprend une partie de circuit chaud et une partie de circuit froid, chacune munie d'un système de pompage et un circuit de retour de fluide.

16. Dispositif selon l'une quelconque des revendications 11 à 15, **caractérisé en ce** le système de transport comprend un ou plusieurs dispositifs de joints (15) dans le conduit de transport, les dispositifs de joints comportant une pluralité de parois flexibles juxtaposées ayant des ouvertures épousant la forme des récipients.

**17.** Dispositif selon la revendication 15 **caractérisé en ce que** la partie de circuit chaud et la partie de circuit froid sont séparées par un ou plusieurs desdits dispositif de joints.

**18.** Dispositif selon l'une quelconque des revendications 11 à 17, **caractérisé en ce que** le système de génération d'impulsions de champ électrique comprend des électrodes arrangées de part et d'autre d'une section de passage du conduit et aptes à générer un champ électrique transversal à cette section.

**19.** Dispositif selon l'une quelconque des revendications 11à 17, **caractérisé en ce que** le système de génération d'impulsions de champ électrique comprend un inducteur avec un ou plusieurs enroulements primaires arrangés de façon toroïdale autour d'une section de passage du conduit et aptes à générer un champ électrique essentiellement longitudinal à cette section.

**20.** Dispositif selon l'une quelconque des revendications 11 à 19, comprenant au moins un capteur de champ électrique dans la zone d'application du champ électrique et des capteurs de température le long du conduit de transport.

**21.** Dispositif selon l'une quelconque des revendications 11 à 20, comprenant une colonne de liquide de transport surplombant la zone de traitement des récipients et ayant une hauteur destinée à générer une pression essentiellement égale à la pression maximale développée à l'intérieur des récipients lors de l'échauffement du liquide à traiter.

**22.** Dispositif selon l'une quelconque des revendications 11 à 20, comprenant des dispositifs de joints dans le conduit de part et d'autre de la station d'échauffement, et un dispositif de pompage créant une pression dans une partie de conduit entre lesdits dispositifs de joint essentiellement égale à la pression maximale développée à l'intérieur des récipients lors de l'échauffement du liquide à traiter.

**Claims**

**1.** Process for the sterilisation or pasteurisation of a liquid to be treated contained in hermetically sealed containers, comprising transport of the containers into a treatment zone where the containers are immersed in a flux of external transport fluid, heating in volume of the liquid to be treated at a speed greater than 28°C per second, to a treatment temperature **T** between 20°C and 66°C, agitation of the container during heating of the liquid, and according to the value of the treatment temperature **T,** exposure of the liquid to an electric field for treatment by electroporation immediately or slightly after heating of the liquid, the amplitude **E** of the electric field in V/cm being selected such that the equation:

$$C(T) \leq \log (E+1) \leq B(T)$$

is satisfied for the values:

$B(T) = -2.340 \times 10^{-5} T^3 + 1.290 \times 10^{-3} T^2 - 3.110 \times 10^{-2} T + 5.0$
$C(T) = -4.503 \times 10^{-5} T^3 + 2.888 \times 10^{-3} T^2 - 5.900 \times 10^{-2} T + 4.0$

where T is the treatment temperature in Celsius.

**2.** The process according to Claim 1, **characterised in that** the electric field for treatment by electroporation alternates with an oscillation frequency of between 100kHz and 1000 kHz, and is supplied in pulses.

**3.** The process according to Claim 2 **characterised in that** the total calorific energy supplied to the liquid to be treated by said electric field pulse or pulses is less than $0.05J/cm^3$.

**4.** The process according to Claim 2 or 3 **characterised in that** the duration of application of a pulse of the electric field is between 10 and 100 microseconds and the frequency of repetition of the electric field pulses is between 10 and 100Hz.

**5.** The process according to any one of the preceding claims, **characterised in that** application of the electric field for treatment by electroporation is carried out after the heating step of the liquid followed by a pause during which the electric field is zero or negligible.

6. The process according to any one of the preceding claims, **characterised in that** the heating speed is greater than 30°C per second.

7. The process according to any one of the preceding claims, **characterised in that** the transport fluid is water or a water-based liquid.

8. The process according to Claim 7, **characterised in that** the transport liquid is turbulised in rotation around the containers.

9. The process according to any one of the preceding claims **characterised in that** the static pressures developed in the treatment zones are created by pumping and chamber systems.

10. The process according to any one of the preceding claims, **characterised in that** the static pressures developed in the treatment zone are created by columns of liquid constituting the external fluid rising above the container treatment zone.

11. A device for carrying out a process for the sterilisation or pasteurisation of a liquid to be treated which is water-based or contains water contained in hermetically sealed containers, comprising a transport system (2) of the liquid to be treated (3), a station for heating in volume (4) the liquid to be treated comprising a wave generator operating at a frequency greater than 1MHz and a station for application of an electric field in pulses (5), the transport system comprising a transport conduit (7, 7') in which a flux of transport fluid circulates, and means for agitation or turbulisation of the transport fluid in the proximity of the heating station, the heating system being configured to heat the liquid in the containers passing through the heating station at a treatment temperature T between 20°C and 66°C at a rate greater than 28°C per second, and the station for application of an electric field in pulses (5) being configured to generate a electric field for treatment by electroporation immediately or slightly after heating of the liquid, of amplitude E in V/cm such that the equation:

$$C(T) \leq \log (E+1) \leq B(T)$$

is satisfied for the values:

$B(T) = -2{,}340 \times 10^{-5} \, T^3 + 1{,}290 \times 10^{-3} \, T^2 - 3{,}110 \times 10^{-2} \, T + 5.0$
$C(T) = -4{,}503 \times 10^{-5} \, T^3 + 2{,}888 \times 10^{-3} \, T^2 - 5{,}900 \times 10^{-2} \, T + 4.0$

where T is the treatment temperature in Celsius.

12. The device according to the preceding claim, **characterised in that** the station for generation of electric fields by pulses is configured to generate an alternating electric field with an oscillation frequency of between 100kHz and 1000 kHz in pulses with a duration between 10 and 100 microseconds.

13. The device according to Claim 12 **characterised in that** the system for generating electric field by pulses is configured to supply a total calorific energy of less than 0.05 J/cm$^3$ to the liquid to be treated.

14. The device according to any one of Claims 11 to 13, **characterised in that** the transport fluid is water or a water-based liquid.

15. The device according to any one of Claims 11 to 14, **characterised in that** the transport system comprises a hot circuit part and a cold circuit part, each having a pumping system and a fluid return circuit.

16. The device according to any one of Claims 11 to 15, **characterised in that** the transport system comprises one or more seal devices (15) arranged in the transport conduit, the seal devices comprising a plurality of juxtaposed flexible walls having openings fitting to the form of the containers.

17. The device according to any one of Claim 15, **characterised in that** the hot circuit part and cold circuit part are separated by one or more of said seal devices.

18. The device according to any one of Claims 11 to 17, **characterised in that** the system for generation of electric field pulses comprises electrodes arranged on either side of a section of passage of the conduit and capable of generating an electric field transversal to this section.

19. The device according to any one of Claims 11 to 17, **characterised in that** the system for generation of electric field pulses comprises an inductor with one or more primary windings arranged toroidally about a section of passage of the conduit and capable of generating an electric field essentially longitudinal to this section.

20. The device according to any one of Claims 11 to 17, comprising at least one electric field sensor in the zone of application of the electric field and temperature sensors along the transport conduit.

21. The device according to any one of Claims 11 to 20, comprising a transport liquid column rising above the container treatment zone and having a height for generating a pressure essentially equal to the maximum pressure developed inside containers during the heating of the liquid to be treated.

22. The device according to any one of Claims 11 to 20, comprising seal devices in the conduit on either side of the heating station, and a pumping device creating pressure in a part of the conduit between said seal devices essentially equal to the maximum pressure developed inside containers during heating of the liquid to be treated.


**Patentansprüche**

1. Verfahren zur Sterilisierung oder Pasteurisierung einer zu behandelnden Flüssigkeit die in hermetisch verschlossenen Behältern enthalten ist, unfassend : den Transport der Behälter in einem Behandlungsbereich wo die Behälter in ein äußeres Transportflussfluid getaucht werden, die volumenmäßige Erhitzung der zu behandelnden Flüssigkeit mit einer Geschwindigkeit von über 28°C je Sekunde bis auf eine Behandlungstemperatur T, die zwischen 20°C und 66°C liegt, das Bewegen des Behälters beim Erhitzen der Flüssigkeit und, gemäß dem Wert der Behandlungstemperatur T, die Exposition der Flüssigkeit gegenüber einem elektrischen Feld zur Behandlung durch Elektroporation unmittelbar oder kurz nach dem Erhitzen der Flüssigkeit, wobei die Amplitude E des elektrischen Feldes in V/cm derart ausgewählt ist, dass die Gleichung:

$$C(T) \leq \log(E+1) \leq B(T)$$

erfüllt wird für die Werte:

$B(T) = -2,340 \cdot 10^{-5} T^3 + 1,290 \cdot 10^{-3} T^2 - 3,110 \cdot 10^{-2} T + 5,0$
$C(T) = -4,503 \cdot 10^{-5} T^3 + 2,888 \cdot 10^{-3} T^2 - 5,900 \cdot 10^{-2} T + 4,0$

wobei T die Behandlungstemperatur in Grad Celsius ist.

2. Verfahren nach Anspruch 1, **dadurch gekennzeichnet, dass** das elektrische Feld zur Behandlung durch Elektroporation alternierend ist mit einer Schwingungsfrequenz zwischen 100 kHz und 1000 kHz und in Impulsen geliefert wird.

3. Verfahren nach Anspruch 2, **dadurch gekennzeichnet, dass** die der zu behandelnden Flüssigkeit von dem oder den elektrischen Impulsfeldern insgesamt zugeführte Wärmeenergie kleiner als 0,05 J/cm$^3$ ist.

4. Verfahren nach Anspruch 2 oder 3, **dadurch gekennzeichnet, dass** die Dauer der Anwendung eines elektrischen Impulsfeldes zwischen 10 und 100 Mikrosekunden inklusive ist und die Wiederholungsfrequenz der elektrischen Impulsfelder zwischen 10 und 100 Hz liegt.

5. Verfahren nach einem der vorangehenden Ansprüche, **dadurch gekennzeichnet, dass** die Anwendung des elektrischen Feldes zur Behandlung durch Elektroporation nach dem Schritt des Erhitzens der Flüssigkeit durchgeführt wird, gefolgt von einer Pause, in der das elektrische Feld null oder vernachlässigbar ist.

**6.** Verfahren nach einem der vorangehenden Ansprüche, **dadurch gekennzeichnet, dass** die Geschwindigkeit der Erhitzung höher als 30°C je Sekunde ist.

**7.** Verfahren nach einem der vorangehenden Ansprüche, **dadurch gekennzeichnet, dass** das Transportfluid Wasser oder eine Flüssigkeit auf Wasserbasis ist.

**8.** Verfahren nach Anspruch 7, **dadurch gekennzeichnet, dass** die Transportflüssigkeit rotierend um Behälter verwirbelt wird.

**9.** Verfahren nach einem der vorangehenden Ansprüche, **dadurch gekennzeichnet, dass** die in den Behandlungsbereichen entwickelten statischen Drücke von Pump- und von Schleusensystemen erzeugt werden.

**10.** Verfahren nach einem der vorangehenden Ansprüche, **dadurch gekennzeichnet, dass** die im Behandlungsbereich entwickelten statischen Drücke von den den Behandlungsbereich der Behälter überragenden Flüssigkeitssäulen erzeugt werden, die das äußere Fluid bilden.

**11.** Vorrichtung zur Umsetzung eines Verfahrens zur Sterilisierung oder Pasteurisierung einer zu behandelnden Flüssigkeit auf Wasserbasis oder Wasser enthaltend, die in hermetisch verschlossenen Behältern enthalten ist, umfassend ein Transportsystem (2) der zu behandelnden Flüssigkeit (3), eine Station zur volumenmäßigen Erhitzung (4) der zu behandelnden Flüssigkeit, die einen mit einer Frequenz von über 1 MHz arbeitenden Wellengenerator aufweist, und eine Station zur Anwendung eines elektrischen Impulsfeldes (5), wobei das Transportsystem eine Transportleitung (7, 7') umfasst, in der ein Transportflussfluid zirkuliert, und Mittel zum Bewegen oder Verwirbeln des Transportfluids in der Nähe der Erhitzungsstation, wobei das Erhitzungssystem konfiguriert ist, um die Flüssigkeit in den Behältern zu erhitzen, die die Erhitzungsstation durchqueren, auf eine Behandlungstemperatur T, die zwischen 20°C und 66°C liegt in einem Verhältnis von über 28°C je Sekunde, und wobei die Station zur Anwendung eines elektrischen Impulsfeldes (5) konfiguriert ist, um ein elektrisches Feld zur Behandlung durch Elektroporation der Amplitude E in V/cm unmittelbar oder kurz nach dem Erhitzen der Flüssigkeit zu erzeugen, so dass die Gleichung:

$$C(T) \ \le \ \log(E+1) \ \le \ B(T)$$

erfüllt wird für die Werte:

$B(T) = -2{,}340 . 10^{-5} T^3 + 1{,}290 . 10^{-3} T^2 - 3{,}110 - 10^{-2} T + 5{,}0$
$C(T) = -4{,}503 . 10^{-5} T^3 + 2{,}888 . 10^{-3} T^2 - 5{,}900 - 10^{-2} T + 4{,}0$

wobei T die Behandlungstemperatur in Grad Celsius ist.

**12.** Vorrichtung nach vorangehendem Anspruch, **dadurch gekennzeichnet, dass** die Station zum Erzeugen elektrischer Impulsfelder konfiguriert ist, um ein alternierendes elektrisches Feld mit einer Impuls-Schwingungsfrequenz zu erzeugen, die zwischen 100 kHz und 1000 kHz liegt mit einer Dauer zwischen 10 und 100 Mikrosekunden.

**13.** Vorrichtung nach Anspruch 12, **dadurch gekennzeichnet, dass** das System zur elektrischen Impulsfelderzeugung konfiguriert ist, um der zu behandelnden Flüssigkeit eine Wärmeenergie von insgesamt weniger als 0,05 J/cm$^3$ zuzuführen.

**14.** Vorrichtung nach einem der Ansprüche 11 bis 13, **dadurch gekennzeichnet, dass** das Transportfluid Wasser ist oder eine Flüssigkeit auf Wasserbasis.

**15.** Vorrichtung nach einem der Ansprüche 11 bis 14, **dadurch gekennzeichnet, dass** das Transportsystem einen warmen Kreislaufabschnitt und einen kalten Kreislaufabschnitt umfasst, die jeweils mit einem Pumpsystem und einem Fluidrücklaufkreis ausgestattet sind.

**16.** Vorrichtung nach einem der Ansprüche 11 bis 15, **dadurch gekennzeichnet, dass** das Transportsystem eine oder mehrere Dichtungsvorrichtungen (15) in der Transportleitung umfasst, wobei die Dichtungsvorrichtungen eine Vielzahl nebeneinander liegender flexibler Wände umfassen, die Öffnungen haben, die die Form der Behälter annehmen.

**17.** Vorrichtung nach Anspruch 15, **dadurch gekennzeichnet, dass** der warme Kreislaufabschnitt und der kalte Kreislaufabschnitt durch eine oder mehrere Dichtungsvorrichtungen getrennt sind.

**18.** Vorrichtung nach einem der Ansprüche 11 bis 17, **dadurch gekennzeichnet, dass** das System zum Erzeugen elektrischer Impulsfelder Elektroden umfasst, die auf der einen und der anderen Seite eines Durchgangsabschnitts der Leitung angeordnet sind und imstande, ein zu diesem Abschnitt transversales elektrisches Feld zu erzeugen.

**19.** Vorrichtung nach einem der Ansprüche 11 bis 17, **dadurch gekennzeichnet, dass** das System zum Erzeugen elektrischer Impulsfelder einen Induktor mit einer oder mehreren Primärwicklungen umfasst, die toroidal um einen Durchgangsabschnitt der Leitung angeordnet sind und imstande, ein zu diesem Abschnitt im wesentlichen längs verlaufendes elektrisches Feld zu erzeugen

**20.** Vorrichtung nach einem der Ansprüche 11 bis 19, die mindestens einen elektrischen Feldsensor im Anwendungsbereich des elektrischen Feldes und Temperatursensoren entlang der Transportleitung umfasst.

**21.** Vorrichtung nach einem der Ansprüche 11 bis 20, die eine Flüssigkeitstransportsäule über dem Behandlungsbereich der Behälter umfasst und eine Höhe hat, die dazu bestimmt ist, einen Druck zu erzeugen, der im wesentlichen gleich dem maximalen Druck ist, der sich in den Behältern während der Erhitzung der zu behandelnden Flüssigkeit entwickelt.

**22.** Vorrichtung nach einem der Ansprüche 11 bis 20, die Dichtungsvorrichtungen in der Leitung auf der einen und der anderen Seite der Erhitzungsstation umfasst und eine Pumpvorrichtung, die einen Druck in einem Abschnitt der Leitung zwischen den Dichtungsvorrichtungen erzeugt, der im wesentlichen dem maximalen Druck entspricht, der sich in den Behältern während der Erhitzung der zu behandelnden Flüssigkeit entwickelt.

Figure 1

log(E, V/cm)

Echelle Logarithmique

A (T)    B (T)

C (T)

Température en °C

EP 1 972 211 B1

Figure 2

Figure 3

Figure 4b

Figure 4a

Figure 5

EP 1 972 211 B1

Figure 6b

Figure 6a

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4695472 A
- EP 1328167 A
- GB 390768 A
- US 2909436 A
- FR 1436405
- FR 2035678